Europäisches Patentamt

European Patent Office

Office européen des brevets

(11) Veröffentlichungsnummer: **0 102 535**
**A2**

(12) **EUROPÄISCHE PATENTANMELDUNG**

(21) Anmeldenummer: **83107721.9**

(22) Anmeldetag: **05.08.83**

(51) Int. Cl.$^3$: **C 12 P 19/04**
**C 13 K 13/00**

---

(30) Priorität: **10.08.82 DE 3229700**
**11.01.83 DE 3300633**

(43) Veröffentlichungstag der Anmeldung:
**14.03.84 Patentblatt 84/11**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Voelskow, Hartmut, Dr.**
**Eschenstrasse 24**
**D-6234 Hattersheim am Main(DE)**

(72) Erfinder: **Schlingmann, Merten, Dr.**
**Schneidhainer Strasse 32a**
**D-6240 Königstein/Taunus(DE)**

---

(54) Verfahren zur Herstellung von Rhamnose oder Fucose.

㊴ Durch Fermentation mit Bakterien der Gattungen Alcaligenes, Klebsiella, Pseudomonas oder Enterobacter werden extracelluläre Polysaccharide erzeugt, die reich an Rhamnose oder Fucose sind. Diese Desoxyzucker können nach saurer Hydrolyse des Polysaccharids aus dem Hydrolysat isoliert werden.

EP 0 102 535 A2

HOECHST AKTIENGESELLSCHAFT     HOE 83/F 002 J   Dr.KL/mü

Verfahren zur Herstellung von Rhamnose oder Fucose

Die Erfindung betrifft ein Verfahren zur Herstellung von Rhamnose oder Fucose, das dadurch gekennzeichnet ist, daß man durch Fermentation mit Bakterien der Gattungen Alcaligenes, Klebsiella, Pseudomonas oder Enterobacter ein an diesen Desoxyzuckern reiches extracelluläres Polysaccharid erzeugt, dieses hydrolysiert und die gebildeten Desoxyzucker abtrennt.

Die Desoxyzucker Rhamnose und Fucose sind auf chemischem Weg nur sehr schwer zugänglich. Es ist bekannt, daß zahlreiche Bakterien in der Lage sind, Desoxyzucker zu synthetisieren. Lipopolysaccharide enthalten sehr häufig Rhamnose und/oder Fucose, ebenso zahlreiche extracelluläre Polysaccharide, jedoch jeweils nur in sehr geringen Mengen.

Es wurde nun gefunden, daß Bakterien der Gattungen Alcaligenes, Klebsiella, Pseudomonas und Enterobacter extracelluläre Polysaccharide synthetisieren können, die mehr als 10 % Rhamnose und/oder Fucose enthalten, und daß diese Desoxyzucker aus den gebildeten Polysacchariden auch in guten Ausbeuten gewonnen werden können. Im folgenden werden bevorzugte Ausgestaltungen dieser Erfindung näher erläutert.

Eine Ausgestaltung des erfindungsgemäßen Verfahrens besteht darin, ein Bakterium der Gattung Alcaligenes in einem zuckerreichen Medium in belüfteter Submerskultur zu fermentieren, das gebildete Polysaccharid - nach vorheriger Isolierung und gegebenenfalls Trocknung oder unmittelbar - zu hydrolysieren und aus dem Hydrolysat die Rhamnose zu gewinnen.

Eine andere Ausgestaltung der Erfindung besteht darin, daß ein Bakterium der Gattung Pseudomonas, insbesondere der aus der EP-PS 12 552 bekannte Stamm ATCC 31461, wie beschrieben fermentiert und aus dem rhamnosereichen Exopolysaccharid die Rhamnose isoliert wird.

Eine andere Ausgestaltung der Erfindung besteht darin, daß ein Bakterium der Gattung Klebsiella, insbesondere der Art Klebsiella pneumoniae, vor allem der aus der US-PS 4 350 769 bekannte Stamm ATCC 31488, wie beschrieben fermentiert und aus dem fucosereichen Exopolysaccharid die Fucose isoliert wird. Zahlreiche nichtpathogene Stämme wurden in letzter Zeit aus der Art K. pneumoniae ausgegliedert und der neuen Art K. planticola zugeordnet. Unabhängig von dieser Nomenklatur betrifft diese Ausgestaltung der Erfindung die Verwendung solcher Bakterien der Gattung Klebsiella, die fucosereiche Exopolysaccharide bilden.

Eine weitere Ausführungsform der Erfindung besteht darin, ein Bakterium der Gattung Enterobacter, vorzugsweise der Arten Enterobacter cloacae oder Enterobacter sakazaki, wie vorstehend beschrieben zu fermentieren und aus dem erhaltenen Polysaccharid Fucose zu gewinnen.

Die Auswahl geeigneter Stämme der genannten Bakterienarten erfolgt durch chromatographische Analyse der gebildeten Exopolysaccharide, die in üblicher Weise hydrolysiert werden, beispielsweise durch Papier-, Dünnschicht-, Hochdruckflüssig- oder Gaschromatographie geeigneter Derivate.

Als zuckerreiche Kulturmedien für die beschriebenen Verfahren dienen solche, die als Kohlenstoffquelle im wesentlichen Glucose, Saccharose, Lactose, Galactose, Fructose oder andere zuckerhaltige Rohstoffe wie Molke enthalten. Als Stickstoffquellen dienen anorganische Salze wie Natriumnitrat oder Ammoniumsalze und/oder organische Stickstoffquellen wie Cornsteep (Maisquellwasser), Hefeextrakt, Sojamehl oder dergleichen. Wird Molke als Zuckerquelle verwendet, so kann aufgrund ihres Eiweißgehaltes der Anteil der übrigen Stickstoffquellen verringert werden. Weiterhin enthalten die Kulturmedien die üblichen Salze und Spurenelemente.

Die Fermentationsbedingungen entsprechen den für Bakterien dieser Gattungen üblichen: Die Temperatur wird in einem Bereich von etwa 25 bis etwa 40°C, insbesondere etwa 30°C gehalten. Der pH-Wert wird auf einen Bereich von etwa 6 bis 7,5, insbesondere etwa 6,8 eingestellt und konstant gehalten. Die Kultur wird mit etwa 1 bis 2 l, vorzugsweise 1,5 l pro Minute und pro l Medium belüftet und gerührt.

Nach etwa 5 bis 7 Tagen ist der Zucker abgebaut und die Bakterien haben etwa 15 bis 25 g Polysaccharid pro Liter Medium, meistens etwa 20 g/l Medium, ausgeschieden. Der Gehalt an Rhamnose bzw. Fucose liegt bei leistungsfähigen Stämmen bei über 20 %, teilweise über 50 %.

Die Hydrolyse der Polysaccharide erfolgt in bekannter Weise, z. B. mit wäßrigen Säuren wie Salzsäure, Schwefelsäure, Phosphorsäure oder Essigsäure, zweckmäßig bei erhöhter Temperatur, vorzugsweise bei etwa 80 bis 120°C.

Die Hydrolyse ist im allgemeinen nach 4 bis 10 Stunden abgeschlossen. Je nach Art der eingesetzten Säure kann diese durch Destillation oder neutralisierende Fällung entfernt und die säurefreie zuckerhaltige Lösung in bekannter Weise aufgetrennt werden.

Je nach Zusammensetzung des Hydrolysats erfolgt die Abtrennung des gewünschten Desoxyzuckers durch chromatographische Methoden, beispielsweise durch Ionenaustauschchromatographie, oder Adsorption an geeigneten oberflächenreichen Substanzen wie Zeolithen. Eine andere bevorzugte Aufarbeitung besteht darin, daß das Hydrolysat fermentativ aufgearbeitet wird, wobei die Nebenprodukte abgebaut werden und der gewünschte Desoxyzucker angereichert wird.

Die erfindungsgemäß erhältlichen Methylpentosen Rhamnose und Fucose eignen sich als Ausgangsstoff für die Synthese von Aromastoffen.

In den folgenden Beispielen beziehen sich Prozentangaben auf das Gewicht, sofern nichts anderes angegeben ist.

Beispiel 1

Alcaligenes sp. wird in einem Kulturmedium gezüchtet, das pro Liter die folgenden Inhaltsstoffe enthielt:

| 50 | g Glucose | 0,005 | g Fe(III)citrat |
|---|---|---|---|
| 1,5 | g Cornsteep (trocken) | 0,001 | g $MnSO_4$ |
| 1,0 | g $NaNO_3$ | 0,0005 | g $ZnCl_2$ |
| 1,0 | g $KH_2PO_4$ | 0,00012 | g $CuSO_4$ |
| 0,5 | g $MgSO_4 \cdot 7H_2O$ | 0,00011 | g $CoCl_2$ |
| 0,015 | g $CaCl_2$ | 0,00012 | g $Na_2MoO_4 \cdot 2H_2O$ |

Die Temperatur der Kultur wird auf 30°C und der pH-Wert auf 6,8 eingestellt und konstant gehalten. Die gerührte Kultur wird mit 1,5 l Luft pro Minute und pro Liter Medium versorgt. Nach 6 Tagen ist der Zucker abgebaut.

Pro Liter Kulturmedium wurden etwa 20 g Polysaccharid ausgeschieden, das zu etwa 50 % aus Rhamnose, 30 % Glucose, 15 % Galactose sowie etwa 5 % Fucose bestand.

Das Polysaccharid wurde mit wäßriger Salzsäure bei etwa 100°C im Verlauf von 6 Stunden hydrolysiert. Anschließend wurde der Chlorwasserstoff durch Destillation entfernt.

Das neutrale Hydrolysat wird auf eine Zuckerkonzentration von etwa 20 % mit Wasser verdünnt und 1 % Hefeextrakt, 0,6 % Harnstoff, 0,12 % $KH_2PO_4$ und 0,018 % $Na_2HPO_4$ zugesetzt. Nach Einstellung des pH-Wertes auf 6,0 bis 6,5 wird mit einer Hefe der Art Saccharomyces cerevisiae beimpft, die die Glucose und Galactose aus dem gespaltenen Polysaccharid unter anaeroben Bedingungen bei 30 bis 37°C zu Ethanol vergärt und die Desoxyzucker zurückläßt.

Im Kulturmedium kann anstelle der Glucose als Zucker auch Saccharose, Lactose, Galactose, Mannose, Fructose oder Molke eingesetzt werden, wobei im Falle der Molke nach Bestimmung des Lactosegehalts ebenfalls etwa 50 g Zucker pro Liter eingesetzt werden. Aufgrund des Eiweißgehaltes der Molke wird der Anteil an Cornsteep und $NaNO_3$ entsprechend reduziert. Man erhält so zwischen 15 und 25 g Polysaccharid pro Liter, die zu 40 bis 60 % aus Rhamnose, 20 bis 40 % aus Glucose, 10 bis 20 % aus Galactose sowie 1 bis 5 % Fucose bestehen.

Die Hydrolyse kann anstelle von Salzsäure auch mit Schwefelsäure, Phosphorsäure oder Essigsäure bei 80 bis 120$^{o}$C im Verlauf von 4 bis 10 Stunden erfolgen. Nach der Hydrolyse kann die Essigsäure - wie die Salzsäure - durch Destillation entfernt werden, während Schwefelsäure und Phosphorsäure mit Hilfe von Calciumionen abgetrennt werden können.

Die Abtrennung der Glucose und Galactose aus dem Hydrolysat durch Vergärung kann anstelle von Saccharomyces cerevisiae auch mit anderen Hefearten, einzeln oder gemeinsam, erfolgen.

Aus dem Hydrolysat kann die Glucose auch durch das Enzym Glucoseoxydase in Gegenwart von Sauerstoff in Gluconsäure überführt werden, die bei pH 11 bis 12 mit Calcium- oder Magnesium-Ionen in Form der schwerlöslichen Salze abgetrennt werden kann. Hierbei bleiben die nicht umgesetzten Zucker im Überstand gelöst, woraus sie isoliert und, falls erforderlich, nach bekannten Methoden getrennt werden können.

Beispiel 2

Beispiel 1 wird in der Form abgewandelt, daß das Kulturmedium anstelle von $NaNO_3$ und $KH_2PO_4$ pro Liter 1 g Hefeextrakt und anstelle der 1,5 g Cornsteep 2 g enthält. Weiterhin wird anstelle von Alcaligenes sp. Enterobacter sakazaki eingesetzt. Die Fermentationsparameter stimmen mit Beispiel 1 überein.

In der Kultur werden nach 6 Tagen 16 g Polysaccharid pro Liter gebildet, wobei die Glucose zu 95 % abgebaut ist. Das Polysaccharid enthält etwa 30 % Fucose.

Arbeitet man anstelle von Glucose mit den anderen in Beispiel 1 genannten Zuckern bzw. Molke, so erhält man innerhalb von 5 bis 7 Tagen pro Liter Kulturmedium 12 bis 18 g Polysaccharid, das 20 bis 40 % Fucose enthält.

Beispiel 3

Das nach Beispiel 1 der EP-PS 12 552 erhaltene rhamnosereiche Exopolysaccharid wird gemäß Beispiel 1 aufgearbeitet und die Rhamnose isoliert.

Beispiel 4

Nach den Angaben der US-PS 4 350 769 wird ein fucosereiches Exopolysaccharid erzeugt und gemäß Beispiel 1 aufgearbeitet.

Patentansprüche:

1. Verfahren zur Herstellung von Rhamnose oder Fucose, dadurch gekennzeichnet, daß man durch Fermentation mit Bakterien der Gattung Alcaligenes, Klebsiella, Pseudomonas oder Enterobacter ein an diesen Desoxyzuckern reiches extracelluläres Polysaccharid erzeugt, dieses hydrolysiert und die gebildeten Desoxyzucker abtrennt.

2. Verfahren nach Anspruch 1, dadurch gekennzeichnet, daß man mit Bakterien der Gattung Alcaligenes oder Pseudomonas ein rhamnosereiches Polysaccharid erzeugt.

3. Verfahren nach Anspruch 1 oder 2, dadurch gekennzeichnet, daß Bakterien des Pseudomonas-Stammes ATCC 31 461 eingesetzt werden.

4. Verfahren nach Anspruch 1, dadurch gekennzeichnet, daß man mit Bakterien der Gattung Enterobacter oder Klebsiella ein fucosereiches Polysaccharid erzeugt.

5. Verfahren nach Anspruch 4, dadurch gekennzeichnet, daß Bakterien der Art Enterobacter sakazaki oder cloacae eingesetzt werden.

6. Verfahren nach Anspruch 4, dadurch gekennzeichnet, daß Bakterien der Art Klebsiella pneumoniae eingesetzt werden.

7. Verfahren nach Anspruch 6, dadurch gekennzeichnet, daß Bakterien des Stammes ATCC 31 488 eingesetzt werden.

8. Verfahren nach einem oder mehreren der Ansprüche 1 bis 7, dadurch gekennzeichnet, daß die Fermentation unter aeroben Bedingungen bei 25 bis 40°C und in einem pH-Bereich von 6 bis 7,5 durchgeführt wird und das gebildete Polysaccharid bei erhöhter Temperatur mit Säuren hydrolysiert wird.

9. Verfahren nach Anspruch 8, dadurch gekennzeichnet, daß die Hydrolyse bei 80 bis 120°C erfolgt.

10. Verfahren nach einem oder mehreren der Ansprüche 1 bis 9, dadurch gekennzeichnet, daß die Abtrennung des gebildeten Desoxyzuckers durch Chromatographie, Adsorption oder fermentativen Abbau der Nebenprodukte erfolgt.